# EUROPEAN PATENT SPECIFICATION

(11) **EP 2 187 759 B1**
(45) Date of publication and mention of the grant of the patent: **24.10.2012**
(21) Application number: 08802261.1
(22) Date of filing: 16.09.2008
(51) Int. Cl.: A23G 3/34, A23G 3/38, A23G 3/42, A61K 9/28

(54) **ERYTHRITOL-BASED HARD COATINGS**
HARTBESCHICHTUNGEN AUF ERYTHRITOLBASIS
REVÊTEMENTS DURS À BASE D'ÉRYTHRITE

(30) Priority: 21.09.2007 EP 07018623
(43) Date of publication of application: 26.05.2010
(73) Proprietor: Cargill, Incorporated, Wayzata, MN 55391-5624 (US)
(72) Inventor: GONZE, Michel, Henri, André, B-1020 Bruxelles (BE); VERCAUTEREN, Ronny, Leontina, Marcel, B-9120 Beveren (BE)
(74) Representative: Wibbelmann, Jobst
(86) International application number: PCT/EP2008/007732
(87) International publication number: WO 2009/036954

(56) References cited:
- EP-A- 0 753 262
- WO-A-01/95738
- WO-A-95/07625
- DATABASE WPI Week 200442 Thomson Scientific, London, GB; AN 2004-444077 XP002469428 & JP 2004 137224 A (NIKKEN CHEM CO LTD) 13 May 2004 (2004-05-13)

## Description

The present invention relates to a coated product comprising a core and a hard coating surrounding the core, wherein the hard coating is composed of at least one coating layer, which comprises erythritol and one or more crystallization modifiers and, a process for preparing such a coated produce.

Edible products are often enclosed with hard or soft coatings, which allow to improve the visual appearance of or to confer a pleasant taste to a product, to preserve the taste of an edible food for a longer period of time, to maintain a certain moisture content, and to provide a barrier for unpalatable ingredients or for unpleasant odours, which escape from the coated product and/or impair the taste of the coated product.

Hard coatings are conventionally prepared by using panning procedures, which typically work with sucrose. In recent years, advances in coating technique, such as panning, have allowed the use of other carbohydrate materials to be used in the place of sucrose. In particular, efforts have been devoted to developing sugar-free hard coatings for use in edible products since the typically used sugars, such as sucrose, proved to be detrimental for teeth and causes dental caries due to the formation of acids in the oral cavity. Therefore, great efforts have been directed to the development of coatings for edible products that are made of compositions containing sugar substitutes, in particular containing those sugar substitutes that belong to the class of polyol compounds, such as xylitol, sorbitol, lactitol, maltitol, mannitol and erythritol.

One of the compounds that have been suggested as a substitute for sugar in hard coatings is xylitol (see, e.g., US 4,681,766, US 4,786,511 and US 4,828,845). A drawback of xylitol, however, is that it is relatively expensive compared to other polyols that are suited as sugar substitutes.

Sorbitol is another known sugar substitute and one of the most inexpensive polyols. Therefore, considerable efforts were directed to prepare hard coatings using sorbitol to replace at least some of the rather expensive xylitol (see, e.g. US 5,536,511). However, the use of sorbitol is hampered by the fact that it is hygroscopic in nature and does not readily crystallize. A number of methods have been developed for the coating of sorbitol (see, e.g., US 4,238,510, and US 4,423,086) or mixtures of sorbitol and xylitol (see, e.g., US 5,536,511). However, the properties of the obtained sorbitol containing coating layers have never been satisfactory. Some of the drawbacks that have been observed in connection with sorbitol coatings are an uneven distribution, a rough surface and an unsatisfactory crunchiness, which may all be attributed to crystallization difficulties that affect the appearance of the final coating, as well as the absence of a cooling effect and the lack of a cost-effective advantage due to the incorporation of only low amounts of sorbitol.

Other polyols that have been used as sugar substitutes in the preparation of hard coatings include lactitol and maltitol (see, e.g., US 4,840,797). However, these polyols need to have a high purity in order to obtain an even crystallization and good quality coatings. A further polyol that may also be used for the preparation of a hard coating is mannitol. However, the use of pure mannitol may be associated with problems in the panning process since the solubility of mannitol is relatively low and, thus, too much of the solvent, such as water, would have to be evaporated.

Another known sugar substitute is erythritol, a natural sugar alcohol that has been approved for use as a sweetener throughout the world. Erythritol is a tetrahydric polyol having the structural formula HOCH₂-CHOH-CHOH-CH₂OH and is commercially available as a non-hygroscopic crystalline powder. It does not affect blood sugar, does not contribute to tooth decay (dental caries), does not significantly contribute to calories and unlike some other polyols does not cause gastric distress due to its ability to be absorbed by the body. In addition, erythritol is known for its cooling effect.

WO 95/07625 discloses a chewing gum product, which comprises a gum pellet covered by a hard coating containing erythritol. Preferably, the hard coating consists of a co-crystallized mixture of 20% to about 60% by weight erythritol and 40% to about 80% by weight xylitol. However, the appearance of these coatings is often affected by crystallization problems resulting in coatings that have been reported to easily peel off from the core or to have a rough surface or other surface defects. It was further observed that a high quantity of erythritol, i.e. a weight proportion of erythritol to xylitol of 80:20 and 90: 10, leads to lumpy, rough surfaces, which is probably a result of crystallization difficulties.

EP 1 057 414 A1 describes a hard coating giving good adhesion to cores of edible, chewable or pharmaceutical components, in particular to a chewing gum core. At least one layer of the hard coating comprises a mixture of sorbitol and erythritol, the erythritol being present in an amount of 1 to 50% by weight of the mixture. However, EP 1 057 414 discloses that it is difficult to use more than 50% by weight erythritol since at such high quantities crystallization problems arise which lead to undesirable rough irregular surfaces.

Further, EP 0 753 262 A2 describes a sugar coating composition comprising erythritol, a water-soluble cellulose and an aqueous medium, as well as a solid preparation comprising a center core of a medicine, a food or the like coated with the sugar coating composition.

In addition, WO 01/95738 A1 discloses a method for making coated chewing gum products by coating chewing gum cores with a first coating syrup, which may comprise erythritol as a bulk sweetener and gum arabic, and a second coating syrup comprising a high-intensity sweetener such as aspartame.

In view of the above, the object of the present invention is to provide further coated products having hard coatings based on a sugar substitute, wherein the hard coatings are improved with regard to crystallization and exhibit favourable properties.

This object is solved by the coated product according to claim 1, in which a core is surrounded by at least one coating layer that comprises erythritol and one or more crystallization modifiers, and the process for preparing such a coated product as set forth in claim 6

Thus, according to a first aspect, the present invention relates to a coated product comprising a core and a hard coating surrounding the core, wherein the hard coating includes at least one coating layer, which comprises erythritol and one or more crystallization modifiers.

The erythritol constitutes at least 85% by weight, preferably 90% by weight, of the at least one coating layer, whereas the crystallization modifier (i) constitutes up to 10% by weight, preferably up to 5% by weight, more preferably up to 2% by weight, of the at least one coating layer and is selected from a group (A) consisting of microbial gums, agar agar, pectin, alginic acid, sodium alginate, beta-glucans, carrageenan, glucomannan, guar gum, gum ghatti, gum tragacanth, karaya gum, tara gum, fenugreek gum, locust bean gum, and mixtures thereof, in particular mixtures of two or more thereof, or (ii) constitutes up to 15% by weight, preferably up to 10% by weight, of the at least one coating layer and is selected from a group (B) consisting of maltitol, starch, starch derivatives, hydrolyzed fibers, partially hydrolyzed fibers, water-soluble dietary fibers, glycerol, gluconic acid and gluconic acid derivatives, glucaric acid and glucaric acid derivatives, glucuronic acid and glucuronic acid derivatives, mono-, di- and triglycerides and derivatives thereof, phospholipids, fatty acid esters of non-glycerol polyols or monovalent alcohols, esters of mono-, di- or tricarboxylic acids with fatty acids, mono-, di- or oligosaccharides, sugar-esters, and mixtures thereof, or (iii) constitutes up to 15% by weight, preferably up to 10% by weight, of the at least one coating layer and is a combination of at least one compound of group (A) and at least one compound of group (B). If only a sugar-ester is used as the crystallization modifier, it may be any sugar-alcohol, but not erythritol. Among the crystallization modifiers mentioned above, vegetable gums are especially suited for use within the present invention.

It has been unexpectedly and surprisingly found that the crystallization modifiers described herein allow controlling the crystallization of an erythritol-based hard coating, which is essential in obtaining good hard coatings. More specifically, the crystallization modifiers used within the context of the present invention result in a controlled and even crystallization. In addition, the crystallization modifiers act and can be used as retardants of the kinetics of crystallization. These crystallization modifiers enable the preparation of sugar-free or sugar-reduced erythritol-based hard coatings that adhere well to a given core and exhibit desirable coating properties, such as crunchiness. In addition, the erythritol used in the hard coatings of the present invention provides a desirable cooling effect. The hard coatings of the present invention are therefore suitable for a variety of applications, for example for coating pharmaceutical preparations and chewable or edible products.

The term "vegetable gums", as used herein, includes all plant-derived gum polysaccharides, such as agar agar, pectin, alginic acid, sodium alginate, beta-glucans, carrageenan, glucomannan, guar gum, gum ghatti, gum tragacanth, karaya gum, tara gum, fenugreek gum and locust bean gum. Preferred examples of vegetable gums in the context of the present invention are guar gum, locust bean gum, beta-glucans, alginates, and carrageenan.

A particular preferred vegetable gum for use herein is guar gum, which is defined as a galactomannan consisting of a α-(1,4)-linked β-D-mannopyranose backbone with branch points from their 6-positions linked to α-D-galactose. It is non-ionic and typically made up of about 10,000 residues. Guar gum is highly water-soluble and, for example, more soluble than locust bean gum.

Another preferred vegetable gum for use within the present invention is locust bean gum, which is a galactomannan similar to guar gum. It is polydisperse, non-ionic, and is made up of about 2,000 residues. Locust bean gum is less soluble and less viscous than guar gum and needs heating to dissolve but is soluble in hot water.

A further preferred vegetable gum according to the present invention is any polysaccharide belonging to the class of beta-glucans, which are defined to consist of linear unbranched polysaccharides of linked β-(1,3)-D-glucopyranose units in a random order. Beta-glucans occur, for example, in the bran of grains, such as barley, oats, rye and wheat.

A yet further preferred vegetable gum within the context of the present invention is alginate, a group of linear polymers containing β-(1,4)-lined D-mannuronic acid and α-(1,4)-linked L-guluronic acid residues produced by seaweeds.

A still further preferred vegetable gum for use herein is carrageenan. Carrageenan is a generic term for polysaccharides prepared by alkaline extraction from red seaweed. Carrageenan includes linear polymers of about 25,000 galactose derivatives. The basic structure of carrageenan consists of alternating 3-linked β-D-galactopyranose and 4-linked α-D-galactopyranose units. There are three main classes of commercial carrageenan: the kappa, Iota and lambda carrageenan.

The term "microbial gums", as used herein, is intended to mean all gum polysaccharides of microbial origin, i.e. from algae, bacteria or fungi. Examples thereof include, for example, gellan and xanthan gums that are both produced by bacteria. A preferred microbial gum for use herein is xanthan gum, a microbial desiccation resistant polymer prepared commercially by aerobic submerged fermentation. Xanthan is an anionic polyelectrolyte with a β-(1,4)-D-glucopyranose glucan backbone having side chains of (3,1)-α-linked D-mannopyranose-(2,1)-β-D-glucuronic acid-(4,1)-β-D-mannopyranose on alternating residues.

Within the context of the present invention, the term "starch" denotes a mixture of two molecular entities, namely amylose and amylopectin. Amylose is the starch polysaccharide that primarily consists of long chained α-1,4-linked D-glucose molecules with a degree of polymerization (DP) of typically about 500 to 5,000. Amylopectin consists of relatively short chain α-1,4-linked D-glucose molecules interconnected by many α-1,6-branch points. The molecular weight of amylopectin molecules is typically in the range of several millions. The amylopectin to amylose ratio can vary between 100:0 and 10:90 depending on the plant source. Typical commercial starch sources that may be used within the present invention are maize, waxy maize, high amylose maize, wheat, potato, tapioca, rice, pea and sago. Starches are typically organized in the form of cold water insoluble granules with a diameter usually ranging from 0.5 µm to about 1.0 µm.

In addition to starch also "starches derivatives" or "modified starches" may be used in the context of the present invention. Starch derivatives or modified starches within the meaning of the present invention are starch products whose properties have been altered by physical or chemical means or by the introduction of substituents and whose granular and molecular structures, respectively, are more or less retained. Chemical modification can include esterification or etherification and oxidation reactions at the hydroxyl groups at carbon atoms 2, 3 and 6 of the D-glucose residues. Typical substituents at the hydroxyl groups in modified starches are acetyl, n-octenyl succinate, phosphate, hydroxypropyl, or carboxymethyl groups. Furthermore, the modification can also lead to the formation of cross-links by appropriate substituents, such as phosphate, adipate or citrate. These chemical modifications can be followed by scission of the glucosidic α-1,4 and α-1,6 bonds. Such a partial degradation of starch is usually obtained by treatment with acids, oxidizing agents or with hydrolytic enzymes. Finally, the modified starches also may include native or modified starches that have been converted into a cold-water dispersible form by a heat-moisture treatment followed by drying (e.g. drum drying or spray-cooking). Preferably acid thinned hydroxypropyl tapioca starch is used as starch derivative.

The terms "hydrolyzed fibers" and "partially hydrolysed fibers", as used herein, include any oligosaccharides resulting from the hydrolysis or partial hydrolysis of dietary fibers and having a molecular weight of less than 10,000 and an average degree of polymerization (DP) of 2 to 40, in particular 3 to 30. An example of such a hydrolyzed fiber or partially hydrolyzed fiber are dextrins, which are low-molecular weight carbohydrates produced by the hydrolysis of starch.

The term "water-soluble dietary fibers" as used herein, include all indigestible fiber components of plant foods that are water-soluble and that consist of non-starch polysaccharides. Typical examples of water-soluble dietary fibers for use herein are chitins and fructans, as well as vegetable and bacterial gums.

A particular preferred water-soluble dietary fiber is pectin, which is a heterogenous group of acidic polysaccharides found in fruit and vegetables and mainly prepared from waste citrus peel and apple pomace. Pectin has a complex structure, wherein a large part of the structure consists of homopolymeric partially methylated poly-α-(1,4)-D-galacturonic acid residues with substantial hairy non-gelling areas of alternating α-(1,2)-L-rhamnosyl-α-(1,4)-D-galacturonosyl sections containing branch points with mostly neutral side chains (1 to 20 residues) of mainly L-arabinose and D-galactose. The properties of pectins depend on the degree of esterification, which is normally about 70%. The low-methoxy (LM) pectins are less than 40% esterified, while high-methoxy (HM) pectins are more than 43% esterified, usually 67%.

Another preferred water-soluble dietary fiber are fructans, which are defined as polysaccharides that are composed of fructose units typically having a terminal glucose unit. Specific examples of fructans are inulin and levan. Inulin consists of chains of fructose units, which are mostly or exclusively connected to each other by β-(2,1)-linkages. Most of the inulin chains terminate in one glucose unit but the presence of said glucose unit is not necessary. Levan consists of chains of fructose units, which are mostly or exclusively connected to each other by β-(2,6)-linkages. A terminal glucose unit may be present or not.

A further suitable crystallization modifier for use herein is gluconic acid, which is meant to include any salt thereof, such as sodium gluconate, as well as gluconic acid derivatives, such as gluconic acid lactone. Glucaric acid may also be used and is to be understood to include any salt thereof, such as mono potassium glucarate, as well as glucaric acid derivatives, such as esters or lactones of glucaric acid. In addition, other suitable crystallization modifiers for use herein include glucuronic acid, which is meant to include all salts thereof, such as sodium glucuronic acid, and glucuronic acid derivatives, such as glucuronolacton.

The term "mono-, di- and triglycerides and derivatives thereof", as used herein, includes, inter alia, glycerol esters of saturated or unsaturated fatty acids having 10 to 30, preferably 14 to 22, more preferably 16 to 18, carbon atoms. The term "triglyceride", as used herein, is also meant to include natural vegetable oils or fats, such as cocoa butter. A derivative of mono-, di- and triglycerides is, for example, citric acid esters of mono- and diglycerides of fatty acids.

The term "phospholipids", as used herein, is meant to include phosphoglycerides and sphingomyelins. Preferred for use in the present invention are phosphoglycerides, i.e. phosphatidate or phosphoesters thereof. A preferred example is phosphatidylcholin (lecithin).

Suitable compounds for use herein belonging to the group of "fatty acid esters of non-glycerol polyols" include, for example, mono-, di- or tri-esters of sorbitan with fatty acids. Also suitable for use herein are fatty acid esters of monovalent alcohols. The fatty acid moiety in these compounds may include any branched, straight-chain, unsaturated or saturated fatty acid. Preferably, the fatty acid has 10 to 30 carbon atoms, especially 14 to 22 carbon atoms, and particularly preferred 16 to 18 carbon atoms. The monovalent alcohol has preferably 1 to 10, in particular 1 to 5, carbon atoms.

The term "esters of mono-, di- or tricarboxylic acids with fatty acids" means any ester of a fatty acid, preferably a fatty acid as defined above, with a mono-, di- or tricarboxylic acid, in particular tartaric acid and mono-, di- or tricarboxylic acids of the citric acid cycle.

Preferred disaccharides that may be used within the present invention include isomaltulose, trehalose, sucrose, maltose, cellobiose and lactose. Especially suited are isomaltulose, trehalose, and sucrose. Examples of an "oligosaccharide" suitable for use herein is, for example, oligofructose. If the coating is desired to be sugarless, i.e. free of mono- and disaccharides, the coating layer will not contain mono- or disaccharides.

"Sugar esters" according to the present invention are preferably sucrose esters of fatty acids. Sucrose fatty acid esters are nonionic surfactants consisting of sucrose as hydrophilic group and fatty acid as lipophilic group. Suitable fatty acids for forming the sugar esters are fatty acids having at least 10, preferably at least 12, more preferably at least 14 carbon atoms, and preferably up to 30, more preferably up to 24, and particularly preferred up to 22 carbon atoms. The carbon chains can be linear or branched and saturated or have one or more double bonds. Preferred examples of such fatty acids are stearate, oleate, palmitate, myristate, laurate and the like.

Where the term "comprising" is used in the specification or claims, it is intended to embrace the meaning of "consisting of" or "consisting exclusively of".

According to the present invention, the at least one coating layer may further contain one or more additives, such as artificial sweeteners, for example aspartame and saccharine, dispersing agents, for example titanium dioxide and talc, colouring agents, film formers, for example gelatin, binding agents, and flavouring agents, for example, essential oils or synthetic flavours, as known in the art. Preferably, the coating layer does not contain cellulose or cellulose derivatives and gum arabic.

In a preferred embodiment, the hard coating of the coated product of the present invention comprises more than one coating layer. In a preferred embodiment, the hard coating of the coated product of the present invention is composed of 1 to 100 coating layers, wherein at least one of these coating layers is a coating layer comprising erythritol, one or more crystallization modifiers and, optionally, one or more additives, as defined above (in the following sometimes referred to as "erythritol-based coating layer"). This is to say, that the other coating may comprise one or more erythritol-based coating layers and one ore more layers that are different to the one or more erythritol-based coating layers, wherein the erythritol-based coating layers and the other, different layers may be arranged within the coating in any sequence. Preferably, the coating comprises several, for example 10 to 40, erythritol-based coating layers consecutively disposed upon each other.

Within the coating, the erythritol-based coating layer may be the first layer, which is in direct contact with the core or it may be a layer, which is close enough to the core for effecting good adhesion to the core. For example, the core may be pre-coated with a binder layer of, for example, vegetable gums, maltodextrins, corn syrup, cellulose and cellulose derivatives, starch and starch derivatives and the like, onto which at least one erythritol-based coating layer is applied.

Preferably, the hard coating constitutes from 10 to 80% by weight, more preferably from 20 to 70% by weight, most preferably from 30 to 60% by weight, of the coated product.

The core of the coated product of the present invention is not particularly restricted and may be composed of any coatable material. Preferably, the core is sugar-free. For example, the core may be a product selected from the group consisting of pharmaceutical preparations, such as tablets, chewable products, such as chewing gums, and edible products, such as dietetic products, confectionery products and other food products, such as nuts and dry fruits, for human or animal use. The confectionary products include, for example, chocolate and chocolate containing products, and candies that may be in the form of tablets, lozenges, jellies, chewy pastes and the like.

Preferably, the core constitutes from 20 to 90% by weight, more preferably 30 to 80% by weight, most preferably from 40 to 70% by weight of the coated product.

According to another aspect, the present invention relates to a process for preparing a coated product having a hard coating according to the present invention. This method comprises the following steps:
(a) providing a coating solution, wherein the coating solution comprises a solvent and a coating mixture comprising erythritol and one or more of the above-mentioned crystallization modifiers, and wherein the erythritol constitutes at least 85% by weight of the coating mixture;
(b) coating a plurality of centers in a moving-product coating device by applying the coating solution to the plurality of centers, while moving the plurality of centers by means of the moving-product coating device;
(c) drying the applied coating solution to obtain a coated product.

As regards step (a) of the process, the solvent of the coating solution is typically water. However, a person skilled in the art is able to select other appropriate solvents depending on the coating to be produced and the particular process parameters to be used.

Preferably, the coating solution contains 30 to 85% by weight of the coating mixture and 15 to 70% by weight of the solvent. The coating mixture included in the coating solution comprises 85 to 99.9%, preferably 90 to 99%, more preferably 95 to 98% by weight erythritol and 0.1 to 15%, preferably 1 to 10%, more preferably 2 to 5% by weight of the one or more crystallization modifiers.

The coating mixture may also include low amounts of additives, including artificial sweeteners, dispersing agents, colouring agents, film formers, binding agents and flavouring agents, as described above. In a variation of the process, these additives may also be added prior to or after application of the coating solution to the moving mass of centers. The flavouring agent, for example, may be added to the coating solution or may be applied while the applied coating layer is drying or after the coating layer has been dried.

In step (b), a plurality of centers are coated by applying the coating solution to the plurality of centers, while moving the plurality of centers using a moving-product coating device. The moving-product coating device may be any device that allows to actuate the centers to be coated. Conveniently, the moving-product coating device is a rotating pan. Typically, the coating pan has an ordinary form, i.e. a tulip shape with an inclined axis of rotation or alternatively a cylindrical shape with a horizontal axis. The application of the coating solution to the centers is preferably carried out by spraying an appropriate quantity of the coating solution onto the surface of the moving centers and allowing it to become evenly distributed over the centers. Preferably, the coating solution is supplied to the moving-product coating device or applied to the centers through a heated nozzle.

The centers that are employed in step (b) can be any piece of material independent from its shape and composition, as long as it allows coating at least one erythritol-based coating layer described herein. In particular, the centers can be uncoated cores or pre-coated cores, wherein the cores may be as defined above. The pre-coated cores may already contain one or several coating layers in any sequence that may be the same or different and which may include one or more of the erythritol-based coating layers prepared in accordance with steps (b) and (c) of the process of the present invention. In a preferred embodiment, the center is a core that is pre-coated by a binding layer made of, for example, vegetable gums, maltodextrins, corn syrup, cellulose and cellulose derivatives, starch and starch derivatives and the like.

After having applied the coating solution to the plurality of centers in step (b), the thus obtained coated product is dried in step (c). Conveniently, the drying step may be carried out inside the moving-product coating device by blowing dry and hot air. Preferably, the used drying air has a temperature in the range of 15 to 45°C and/or a moisture content of at most 50%, preferably at most 30%, relative humidity.

In a preferred embodiment, step (b) of coating the plurality of centers and step (c) of drying the applied coating is repeated as many times a desired, either successively or intermittently to allow adding one or more different coating layers. Typically, the coating and drying steps are repeated 1 to 99, usually 1 to 40 or 1 to 25, in particular 1 to 10 times, to build up a plurality of layers. The specific number and nature of the coating layers will depend on the desired application and can be readily determined experimentally by a person skilled in the art.

The coated product obtained by the process of the present invention may be further treated to provide the coated product with certain desirable characteristics, such as physical and organoleptic properties, and/or increase the product's attractivity. For example, a confectionery product may be provided with a gloss coating by glazing, providing the final product with a brilliant surface and a moisture-barrier.

Preferably, the hard coating constitutes from 10 to 80% by weight, more preferably from 20 to 70% by weight, most preferably from 30 to 60% by weight of the final coated product.

As already mentioned, the use of one or more of the crystallization modifiers of the present invention allows to control the crystallization of an erythritol-based coating, leading to an even crystallization and, consequently, to improved properties of the resulting coating layer or coating.

Preferably, the composition and structure of the hard coating is as described above. In particular, the crystallization modifiers are used for preparing coatings of products selected from the group consisting of pharmaceutical preparations, such as tablets, chewable products, such as chewing gums, and edible products, such as dietetic products, confectionery products and other products, such as nuts and dry fruits, for human and animal use. The confectionary products include, for example, chocolate and chocolate containing products, and candies that may be in the form of tablets, lozenges, jellies, chewy pastes and the like. Preferably, the cores as well as the coatings are sugar-free.

The present invention is further illustrated by way of the following Examples.

### EXAMPLE 1

In the experiments described below, the impact of different crystallization modifiers on the crystallization behaviour of erythritol with regard to improving the properties of hard coatings was analyzed.

First, different samples to by analyzed were prepared by dry blending of erythritol with a crystallization modifier in a given ratio, melting the resulting dry blend in an oil-bath set at 155°C, pouring the obtained melt onto an aluminium plate and allowing it to crystallize out, followed by milling the obtained crystallized products. Next, the thus obtained milled products were subjected to a differential scanning calorimetry (DSC) analysis using a Q100 differential scanning calorimeter of TA instruments. Samples were heated twice in a high-pressure sealed crucible from -60°C to 180°C at a heating rate of 5 °C/min. During the controlled cooling from 180°C to -60°C at 5°C/min, the starting crystallization temperature (T_{sc}) and the enthalpy of the crystallization peak (H_{cp}) were measured.

The results of the DSC analysis of erythritol (ErOH) in admixture with a crystallization modifier suitable for use within the present invention as well as erythritol alone as a control are shown in the following Table 1.

**Table 1 Effect of different crystallization modifiers on the starting crystallization temperature (T_{sc}) and the enthalpy of crystallization peak (H_{cp})**

| | T_{sc}[°C] | H_{cp} [J/g] |
|---|---|---|
| ErOH fine (control) | 51.0 | 192.7 |
| ErOH/iota carrageenan UTH18 (97:3 (w/w)) | 31.5 | 156.1 |
| ErOH/mono-diglycerides (98:2 (w/w)) | 28.3 | 150.9 |
| ErOH/gluconic acid lactone (90:10 (w/w)) | 43.9 | 6.9 |
| ErOH/glycerol-triacetate (98:2 (w/w)) | 37.2 | 171.5 |

These results show that the crystallization modifier significantly changes the crystallization behaviour of erythritol and allows controlling the crystallization. Similar effects were observed for other crystallization modifiers, such as guar gum, trehalose, algogel, palm stearin, and isomaltulose.

Thus, the use of the crystallization modifiers described herein allow to control and improve the crystallization behavior of erythritol-based hard coatings and, as a result, enables the preparation of improved hard coatings for a variety of different applications, such as for pharmaceutical products, like tablets, and chewable or edible products.

### EXAMPLE 2

In the experiments described below, the impact of different crystallization modifiers on the crystallization behaviour of erythritol with regard to improving the properties of hard coatings was analyzed in solution.

First, different samples to by analyzed were prepared by blending of erythritol with a crystallization modifier in a given ratio while having a 60 weight solution of erythritol, dissolving the resulting blend in microwave oven. When everything was dissolved, the sample was brought to a water bath at 85°C for 10 minutes. Afterwards the sample was placed on a magnetic stirring plate at 250 rpm at 25°C. A temperature reader was brought in the solution and the temperature was measured.

The results are shown in the following Table 2.

**Table 2 Effect of different crystallization modifiers on the starting crystallization temperature (T_{sc}), the end crystallization temperature (Tₑ), and the crystallization time (t)**

| | T_{sc} [°C] | Tₑ [°C] | t (sec) |
|---|---|---|---|
| ErOH pure (control) | 43.6 | 48.6 | 79 |
| ErOH/kappa carrageenan (99.7:0.3 (w/w)) | 35.8 | 39.9 | 150 |
| ErOH/alginate (Cargill Algogel 5541 (99:1 (w/w)) | 34.3 | 42.9 | 92 |

These results show that the crystallization modifier significantly changes the crystallization behaviour (i.e. crystallization temperature and crystallization time) of erythritol and allows controlling the crystallization.

Thus, the use of the crystallization modifiers described herein allow to control and improve the crystallization behavior of erythritol-based hard coatings and, as a result, enables the preparation of improved hard coatings for a variety of different applications, such as for pharmaceutical products, like tablets, and chewable or edible products.

## Claims

1. A coated product comprising a core and a hard coating surrounding the core, wherein the hard coating includes at least one coating layer, which comprises erythritol and one or more crystallization modifiers, wherein the erythritol constitutes at least 85% by weight of the at least one coating layer, and wherein the crystallization modifier constitutes up to 10% by weight, preferably up to 5%, more preferably up to 2%, of the at least one coating layer and is selected from a group (A) consisting of microbial gums, agar agar, pectin, alginic acid, sodium alginate, beta-glucans, carrageenan, glucomannan, guar gum, gum ghatti, gum tragacanth, karaya gum, tara gum, fenugreek gum, locust bean gum, and mixtures of two or more thereof, or constitutes up to 15% by weight of the at least one coating layer and is selected from a group (B) consisting of maltitol, starch, starch derivatives, hydrolyzed fibers, partially hydrolyzed fibers, water-soluble dietary fibers, glycerol, gluconic acid and gluconic acid derivatives, glucaric acid and glucaric acid derivatives, glucuronic acid and glucuronic acid derivatives, mono-, di- and triglycerides and derivatives thereof, phospholipids, fatty acid esters of non-glycerol polyols or monovalent alcohols, esters of mono-, di- or tricarboxylic acids with fatty acids, mono-, di- or oligosaccharides, sugar-esters, and mixtures thereof, or the crystallization modifier is a combination of at least one compound of group (A) and at least one compound of group (B) in an amount of up to 15% by weight of the at least one coating layer.

2. The coated product according to claim 1, wherein the hard coating comprises 1 to 100 coating layers.

3. The coated product according to claim 1 or 2, wherein the core is selected from the group consisting of pharmaceutical preparations and chewable or edible products.

4. The coated product according to claim 3, wherein the core is a confectionery product.

5. The coated product according to any one of claims 1 to 4, wherein the at least one coating layer further comprises one or more additives selected from the group consisting of artifical sweeteners, dispersing agents, colouring agents, film formers, binding agents, and flavouring agents.

6. A process for preparing the coated product according to any one of claims 1 to 5, comprising:
providing a coating solution, wherein the coating solution comprises a solvent and a coating mixture comprising erythritol and the one or more crystallization modifiers, and wherein the erythritol constitutes at least 85% by weight of the coating mixture;
coating a plurality of centers in a moving-product coating device by applying the coating solution to the plurality of centers, while moving the plurality of centers by means of the moving-product coating device;
drying the applied coating solution to obtain the coated product.

7. The process according to claim 6, wherein the solvent of the coating solution is water.

8. The process according to claim 6 or 7, wherein the coating mixture comprises 85 to 99.9% by weight erythritol and 0.1 to 15% by weight of the one or more crystallization modifiers.

9. The process according to any one of claims 6 to 8, wherein the coating solution contains 30 to 85% by weight of the coating mixture and 15 to 70% by weight of the solvent.

10. The process according to any one of claims 6 to 9, wherein the steps of coating and drying are repeated 1 to 99 times.

11. The process according to any one of claims 6 to 10, wherein the coating solution is applied to the plurality of centers by supplying it through a heated nozzle.

## Patentansprüche

1. Beschichtetes Produkt, umfassend einen Kern und eine den Kern umgebende Hartbeschichtung, wobei die Hartbeschichtung mindestens eine Überzugsschicht einschließt, welche Erythritol und einen oder mehrere Kristallisationsmodifikatoren umfasst, wobei das Erythritol mindestens 85 Gew.-% der mindestens einen Überzugsschicht ausmacht, und wobei der Kristallisationsmodifikator bis zu 10 Gew.-%, vorzugsweise bis zu 5 %, stärker bevorzugt bis zu 2 %, der mindestens einen Überzugsschicht ausmacht und aus der Gruppe (A) gewählt ist, die aus mikrobiellen Gummen, Agar-Agar, Pektin, Alginsäure, Natriumalginat, beta-Glucanen, Carrageenan, Glucomannan, Guargummi, Ghattigummi, Tragant, Karayagummi, Taragummi, Bockshornkleegummi, Johannisbrotgummi und Mischungen von zwei oder mehreren davon besteht, oder bis zu 15 Gew.-% der mindestens einen Überzugsschicht ausmacht und aus der Gruppe (B) gewählt ist, die aus Maltitol, Stärke, Stärkederivaten, hydrolysierten Fasern, teilweise hydrolysierten Fasern, wasserlöslichen Ballaststoffen, Glycerol, Gluconsäure und Gluconsäurederivaten, Glucarsäure und Glucarsäurederivaten, Glucuronsäure und Glucuronsäurederivaten, Mono-, Di- und Triglyceriden und Derivaten davon, Phospholipiden, Fettsäureestern von Nicht-Glycerol-Polyolen oder einwertigen Alkoholen, Estern von Mono-, Di- oder Tricarbonsäuren mit Fettsäuren, Mono-, Di- oder Oligosacchariden, Zuckerestern und Mischungen davon besteht, oder der Kristallisationsmodifikator eine Kombination von mindestens einer Verbindung der Gruppe (A) und mindestens einer Verbindung der Gruppe (B) in einer Menge von bis zu 15 Gew.-% der mindestens einen Überzugsschicht ist.

2. Beschichtetes Produkt gemäß Anspruch 1, wobei die Hartbeschichtung 1 bis 100 Überzugsschichten umfasst.

3. Beschichtetes Produkt gemäß Anspruch 1 oder 2, wobei der Kern aus der Gruppe gewählt ist, die aus pharmazeutischen Präparaten und kaubaren oder essbaren Produkten besteht.

4. Produkt gemäß Anspruch 3, wobei der Kern ein Süßwarenprodukt ist.

5. Beschichtetes Produkt gemäß einem der Ansprüche 1 bis 4, wobei die mindestens eine Überzugsschicht weiter ein oder mehrere Additiv(e) umfasst, das/die aus der Gruppe gewählt ist/sind, die aus künstlichen Süßstoffen, Dispergiermitteln, Färbemitteln, Filmbildnern, Bindemitteln und Aromastoffen besteht.

6. Verfahren für die Herstellung des beschichteten Produkts gemäß einem der Ansprüche 1 bis 5, umfassend:
Vorsehen einer Beschichtungslösung, wobei die Beschichtungslösung ein Lösungsmittel und eine Beschichtungsmischung umfasst, welche Erythritol und den einen oder mehrere Kristallisationsmodifikatoren umfasst, und wobei das Erythritol mindestens 85 Gew.-% der Beschichtungsmischung ausmacht;
Beschichten einer Vielzahl von Zentren in einer Beschichtungsvorrichtung für bewegliche Produkte durch Aufbringen der Beschichtungslösung auf die Vielzahl von Zentren, während die Vielzahl an Zentren mittels der Beschichtungsvorrichtung für bewegliche Produkte bewegt wird;
Trocknen der aufgetragenen Beschichtungslösung für den Erhalt des beschichteten Produkts.

7. Verfahren gemäß Anspruch 6, wobei das Lösungsmittel der Beschichtungslösung Wasser ist.

8. Verfahren gemäß Anspruch 6 oder 7, wobei die Beschichtungsmischung 85 bis 99,9 Gew.-% Erythritol und 0,1 bis 15 Gew.-% von dem einen oder den mehreren Kristallisationsmodifikator(en) umfasst.

9. Verfahren gemäß einem der Ansprüche 6 bis 8, wobei die Beschichtungslösung 30 bis 85 Gew.-% der Beschichtungsmischung und 15 bis 70 Gew.-% des Lösungsmittels enthält.

10. Verfahren gemäß einem der Ansprüche 6 bis 9, wobei die Schritte des Beschichtens und Trocknens 1 bis 99 Mal wiederholt werden.

11. Verfahren gemäß einem der Ansprüche 6 bis 10, wobei die Beschichtungslösung auf die Vielzahl von Zentren durch Zuführen durch eine erhitzte Düse aufgetragen wird.

## Revendications

1. Produit enrobé comprenant un noyau et un enrobage dur entourant le noyau, dans lequel l'enrobage dur inclut au moins une couche d'enrobage, qui comprend de l'érythritol et un ou plusieurs agent(s) de modification de cristallisation, dans lequel l'érythritol constitue au moins 85 % en poids de l'au moins une couche d'enrobage, et dans lequel l'agent de modification de cristallisation constitue jusqu'à 10 % en poids, préférablement jusqu'à 5 %, plus préférablement jusqu'à 2 %, de l'au moins une couche d'enrobage et est choisi parmi un groupe (A) consistant en des gommes microbiennes, l'agar-agar, la pectine, l'acide alginique, l'alginate de sodium, les bêta-glucanes, le carraghénane, le glucomannane, la gomme de guar, la gomme ghatti, la gomme adragante, la gomme karaya, la gomme tara, la gomme de fenugrec, la gomme de caroube, et des mélanges de deux ou plus de ceux-ci, ou bien constitue jusqu'à 15 % en poids de l'au moins une couche d'enrobage et est choisi parmi un groupe (B) consistant en le maltitol, l'amidon, des dérivés d'amidon, des fibres hydrolysées, des fibres partiellement hydrolysées, des fibres alimentaires solubles dans l'eau, le glycérol, l'acide gluconique et des dérivés de l'acide gluconique, l'acide glucarique et des dérivés de l'acide glucarique, l'acide glucuronique et des dérivés de l'acide glucuronique, des mono-, di- et triglycérides et des dérivés de ceux-ci, des phospholipides, des esters d'acides gras de polyols différents du glycérol ou d'alcools monovalents, des esters d'acides mono-, di- ou tricarboxyliques avec des acides gras, des mono-, di- ou oligosaccharides, des esters de sucre, et des mélanges de ceux-ci, ou bien l'agent de modification de cristallisation est une combinaison d'au moins un composé du groupe (A) et d'au moins un composé du groupe (B) dans une quantité jusqu'à 15 % en poids de l'au moins une couche d'enrobage.

2. Produit enrobé selon la revendication 1, dans lequel l'enrobage dur comprend 1 à 100 couches d'enrobage.

3. Produit enrobé selon la revendication 1 ou 2, dans lequel le noyau est choisi parmi le groupe consistant en des préparations pharmaceutiques et des produits à mâcher ou alimentaires.

4. Produit enrobé selon la revendication 3, dans lequel le noyau est un produit de confiserie.

5. Produit enrobé selon l'une quelconque des revendications 1 à 4, dans lequel l'au moins une couche d'enrobage comprend en outre un ou plusieurs additif(s) choisi(s) parmi le groupe consistant en des édulcorants artificiels, des agents de dispersion, des colorants, des agents de formation de film, des agents de liaison, et des aromatisants.

6. Procédé de préparation du produit enrobé selon l'une quelconque des revendications 1 à 5, comprenant :
la prévision d'une solution d'enrobage, dans lequel la solution d'enrobage comprend un solvant et un mélange d'enrobage comprenant de l'érythritol et le ou les plusieurs agent(s) de modification de cristallisation, et dans lequel l'érythritol constitue au moins 85 % en poids du mélange d'enrobage ;
l'enrobage d'une pluralité de centres dans un dispositif d'enrobage de produits en mouvement par application de la solution d'enrobage à la pluralité de centres, tout en déplaçant la pluralité de centres au moyen du dispositif d'enrobage de produits en mouvement ;
le séchage de la solution d'enrobage appliquée pour obtenir le produit enrobé.

7. Procédé selon la revendication 6, dans lequel le solvant de la solution d'enrobage est l'eau.

8. Procédé selon la revendication 6 ou 7, dans lequel le mélange d'enrobage comprend 85 à 99,9 % en poids d'érythritol et 0,1 à 15 % en poids du ou des plusieurs agent(s) de modification de cristallisation.

9. Procédé selon l'une quelconque des revendications 6 à 8, dans lequel la solution d'enrobage contient 30 à 85 % en poids du mélange d'enrobage et 15 à 70 % en poids du solvant.

10. Procédé selon l'une quelconque des revendications 6 à 9, dans lequel les étapes d'enrobage et de séchage sont répétées 1 à 99 fois.

11. Procédé selon l'une quelconque des revendications 6 à 10, dans lequel la solution d'enrobage est appliquée à la pluralité de centres en l'amenant par l'intermédiaire d'une buse chauffée.
